# EUROPEAN PATENT APPLICATION

(11) **EP 4 609 789 A1**
(43) Date of publication of application: **03.09.2025**
(21) Application number: 23882729.9
(22) Date of filing: 26.10.2023
(51) Int. Cl.: A61B 5/256, A61B 5/02, A61B 5/053, A61B 5/11, A61B 5/1455

(54) **BIOSIGNAL MEASUREMENT DEVICE**

(30) Priority: 26.10.2022 JP 2022171713
(71) Applicant: Suntory Holdings Limited, Osaka-shi, Osaka 530-8203 (JP)
(72) Inventor: MIZUTANI, Haruo, Soraku-gun, Kyoto 619-0284 (JP); AZUMI, Hitoshi, Tokyo 108-0075 (JP)
(74) Representative: Vossius & Partner Patentanwälte Rechtsanwälte mbB
(86) International application number: PCT/JP2023/038745
(87) International publication number: WO 2024/090527

(57) **Abstract**

A biosignal measurement device able perform measurement at a suitable portion corresponding to the biosignal to be measured, that is, a biosignal measurement device to be worn by a user, comprising at least one earpiece housing 12 having a biopotential sensor and a board housing 16 having a photoelectric sensor 24 for acquiring a biosignal other than the biopotential as bioinformation, the earpiece housing 12 and board housing 16 being attached at separate portions of the user.

## Description

### FIELD

The present application relates to a biosignal measurement device.

### BACKGROUND

In the medical field, healthcare field, etc., the practice is to measure biosignals and use them to diagnose and manage health. For example, PTL 1 discloses a biosignal detection device provided with a reference electrode configured to be arranged at part of the region comprised of the concha of the auricle and ear canal, a detection electrode configured to contact the skin on the temporal bone behind the auricle, and a signal processing part outputting a signal based on the potential of the reference electrode and the potential of the detection electrode. According to this, it is possible to obtain a significant waveform suitable for analysis of an electroencephalogram.

However, if actively used for health diagnosis or health care, biosignals to be measured generally are diverse in type. Further, sometimes measurements have to be performed continuously over several hours to several days. For this reason, in a device attached to just a single portion of the body such as an ear, like the biosignal detection device described in PTL 1, while the stress of the test subject wearing the device can be kept from increasing, sometimes it is difficult to obtain an overall grasp of the biosignals covered.

### [CITATIONS LIST]]

### [PATENT LITERATURE]

[PTL 1] Japanese Unexamined Patent Publication No. 2018-186934

### SUMMARY

### [TECHNICAL PROBLEM]

The present invention considers the above such problem and has as its object to provide a biosignal measurement device able perform measurement at a suitable portion corresponding to the biosignal to be measured.

### [SOLUTION TO PROBLEM]

One aspect of the present disclosure is a biosignal measurement device to be worn by a user, characterized by comprising at least one biopotential acquiring part having a biopotential sensor and a bioinformation acquiring part having a bioinformation acquisition sensor for acquiring a biosignal other than the biopotential as bioinformation and in that the biopotential acquiring part and the bioinformation acquiring part are attached at separate portions of the user.

Further, in one aspect of the present disclosure, the biopotential acquiring part may be attached to at least one of the back side of an auricle, head, shoulder, and back of the user.

Furthermore, in one aspect of the present disclosure, the bioinformation acquiring part may be attached to the back neck part of the user.

Further, in one aspect of the present disclosure, the biopotential sensor may be configured to be able to measure at least one of an electroencephalogram, electrocardiogram, and electromyogram of the user.

Furthermore, in one aspect of the present disclosure, the biopotential acquiring part may be configured to be able to measure a bioimpedance.

Further, in one aspect of the present disclosure, the bioinformation acquiring part may be configured to be able to measure a blood pressure, plasma glucose, pulse, body surface temperature, and median acceleration.

Furthermore, in one aspect of the present disclosure, the bioinformation acquisition sensor may include a photoelectric sensor.

Further, in one aspect of the present disclosure, the bioinformation acquiring part may be configured to be able to measure at least one of a bioimpedance, temperature, and acceleration.

### [ADVANTAGEOUS EFFECTS OF INVENTION]

According to one aspect of the present disclosure, at least one biopotential acquiring part provided with one biopotential sensor and a bioinformation acquiring part having a bioinformation acquisition sensor for acquiring a biosignal other than the biopotential are attached at separate portions of the user. For analysis and evaluation of the biosignals, the biosignals have to be measured by a significant waveform. They are preferably measured at portions close to the signal sources. Here, since the biopotential sensor and bioinformation acquisition sensor are attached at separate portions, it is possible to respectively attach them to a portion suitable for measurement of the biopotential and a portion suitable for measurement of the bioinformation. Due to this, it is possible to perform suitable measurement corresponding to the biosignal to be measured.

Furthermore, according to one aspect of the present disclosure, the biopotential acquiring part is attached to at least one of the back side of an auricle, head, shoulder, and back of the user. These portions are at positions relatively close to the brain and heart, so are suitable for measurement of the biopotential associated with an electroencephalograms and electrocardiogram and, furthermore, are suitable even for measurement of the biopotential associated with an electromyogram. For this reason, it is possible to perform measurement at suitable portions corresponding to the biosignal to be measured.

Further, according to one aspect of the present disclosure, the bioinformation acquiring part is attached to the back neck part of the user. The back neck part is suited for measuring bioinformation associated with blood since it is near the carotid artery. For this reason, it is possible to suitably measure the biosignal if measuring bioinformation associated with the blood.

Furthermore, according to one aspect of the present disclosure, the biopotential sensor is configured to be able to measure at least one of an electroencephalogram, electrocardiogram, and electromyogram of the user. The biopotential sensor can suitably measure a biosignal relating to an electroencephalogram, electrocardiogram, and electromyogram of the user by being attached to at least one of a back side of an auricle, head, shoulder, or back of the user.

Further, according to one aspect of the present disclosure, the biopotential acquiring part is configured to be able to measure a bioimpedance. A biopotential acquiring part able to measure the bioimpedance generated due to activity of a biological being can also be used to measure the bioimpedance generated by electrically stimulating the biological being. For this reason, it is possible to prevent or keep down an unnecessary increase in the number of sensors and possible to measure a biosignal to be measured after preventing or keeping down an increase in weight of the biosignal measurement device and an increase in the burden on the user accompanying the same.

Furthermore, according to one aspect of the present disclosure, the bioinformation acquiring part is configured to be able to measure a blood pressure, plasma glucose, pulse, body surface temperature, and median acceleration. The bioinformation acquisition sensor can be attached to the back neck part of the user and thereby enables suitable measurement of the blood pressure, plasma glucose, pulse, body surface temperature, and median acceleration.

Further, according to one aspect of the present disclosure, the bioinformation acquisition sensor includes a photoelectric sensor. For this reason, it is possible to suitably measure bioinformation associated with the blood and blood flow such as the blood pressure, plasma glucose, and pulse.

Furthermore, according to one aspect of the present disclosure, the bioinformation acquiring part is configured to be able to measure at least one of a bioimpedance, temperature, and acceleration. For this reason, it is possible to obtain a suitable grasp of correlation of the biosignal with the bioimpedance, temperature, and acceleration when analyzing and evaluating the measured biosignal.

### BRIEF DESCRIPTION OF DRAWINGS

FIG. 1 is a view, seen from the side, of a user to which a biosignal measurement device according to a first embodiment is attached.
FIG. 2(a) is a front view of a bioinformation acquiring part according to the present embodiment, FIG. 2(b) is a plan view of the bioinformation acquiring part, and FIG. 2(c) is a side view of the bioinformation acquiring part.
FIG. 3 is a back view of the bioinformation acquiring part in the state with a back cover removed.
FIG. 4(a) is a view, seen from the side, of a user to which a biosignal measurement device according to a second embodiment is attached and FIG. 4(b) is a view of the user seen from the back side.
FIG. 5(a) is a side view of a bioinformation acquiring part according to the present embodiment, FIG. 5(b) is a plan view of the bioinformation acquiring part, and FIG. 5(c) is a bottom view of the bioinformation acquiring part.

### DESCRIPTION OF EMBODIMENTS

Below, referring to the attached drawings, biosignal measurement devices according to the embodiments will be explained. Similar or corresponding elements will be assigned the same notations and overlapping explanations will be omitted. To facilitate understanding, sometimes the scale of the figures will be changed for the explanation.

### (First Embodiment)

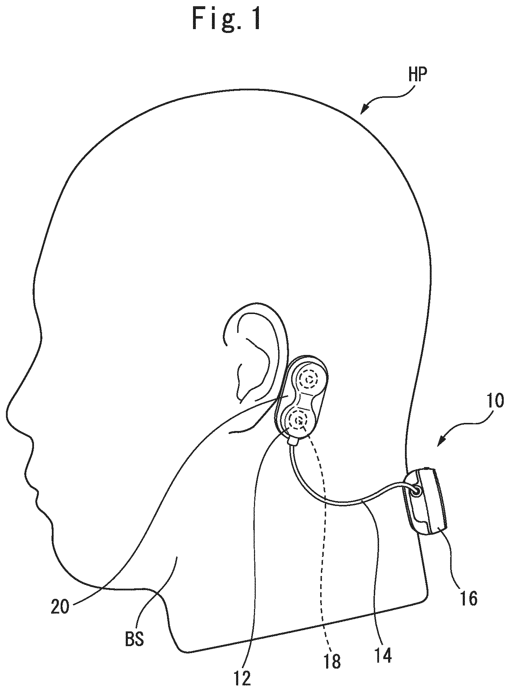

FIG. 1 shows a biosignal measurement device 10 according to a first embodiment attached to a user (measured person) HP. The biosignal measurement device 10 is provided with earpiece housings 12 as biopotential acquiring parts and a board housing 16 as a bioinformation acquiring part connected with the earpiece housings 12 through a harness 14. Here, the earpiece housings 12 are attached to back sides of the auricles at the two left and right sides of the user HP, while the board housing 16 is attached to the back neck part of the user HP. By providing the earpiece housings 12 and board housing 16 dispersed at separate portions of the user HP, it is possible to form them smaller in size and to keep down an increase in weight. Due to this, it is possible to improve the worn feel of the biosignal measurement device 10 to the user.

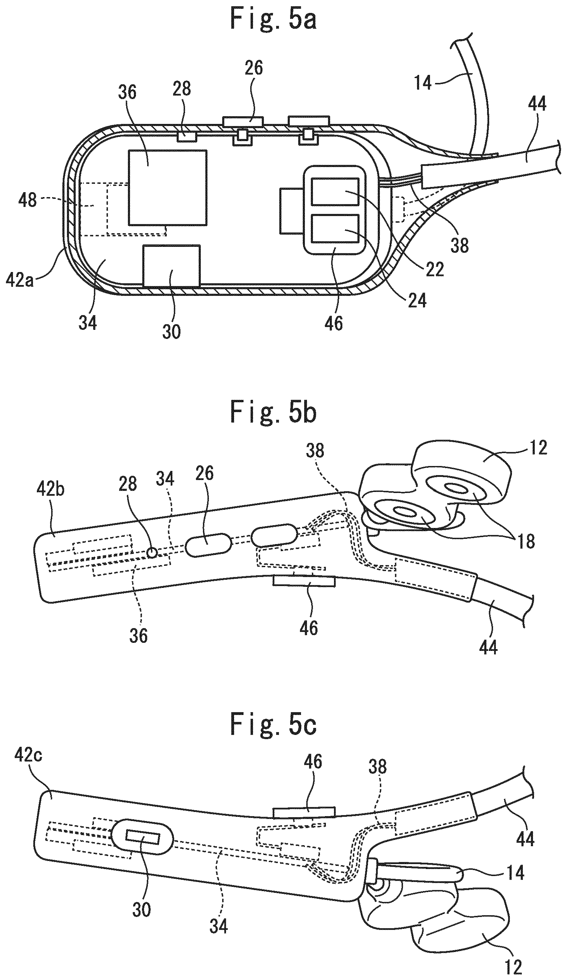

Each earpiece housing 12 has a pair of electrodes 18 (see FIG. 5B) as biopotential sensors attached to the body surface BS for measuring the biopotential generated due activity of the biological being and an attachment part 20 for stably attaching the electrodes 18 to the body surface BS. The pair of electrodes 18 are made lighter in weight by covering them by a cover of a streamlined shape narrowed between the electrodes 18. Only the parts attached to the body surface BS are exposed. The electrodes 18 are formed by conductors. For example, silver, silver chloride, gold, platinum, and other metals may be used. Carbon and other materials may also be used. Here, the electrodes 18 and biosignal measurement device 10 are configured to be able to measure an electroencephalogram (EEG), electrocardiogram (ECG), and electromyogram (EMG). Further, the electrodes 18 are configured to be able to measure bioimpedance by running a weak current from the board housing 16 side. Note that, here, the measurement of the bioimpedance will be explained as being performed by the electrodes 18, but the invention is not limited to this. The configuration for measuring the bioimpedance, that is, the configuration for measuring the impedance by running a weak current, may also be provided at the board housing side.

At the outer circumference of the cover of the electrodes 18, a sheet shaped attachment part 20 with an elliptical shaped outer circumference is formed integrally with the cover of the electrodes 18. The attachment part 20 is configured as a tape with adhesive etc. at the surface of the attachment side. For this reason, it is possible to stably attach the electrodes 18 to the body surface BS. Note that, here, the earpiece housing 12 is explained as comprising electrodes attached to the back side of an auricle of the user HP, but the invention is not limited to this. The earpiece housing may be attached to a portion enabling suitable measurement of the biopotential such as the head, shoulders, and back. Further, here, the earpiece housing 12 used as the biopotential acquiring part is explained as a pair of parts attached to the back sides of auricles of the user HP, but the invention is not limited to this. The biopotential acquiring part may be attached at any place so long as a bodily portion enabling biopotential to be measured such as the two sides of the head, two sides of the shoulders, and/or two sides of the back in addition to the earpiece housing.

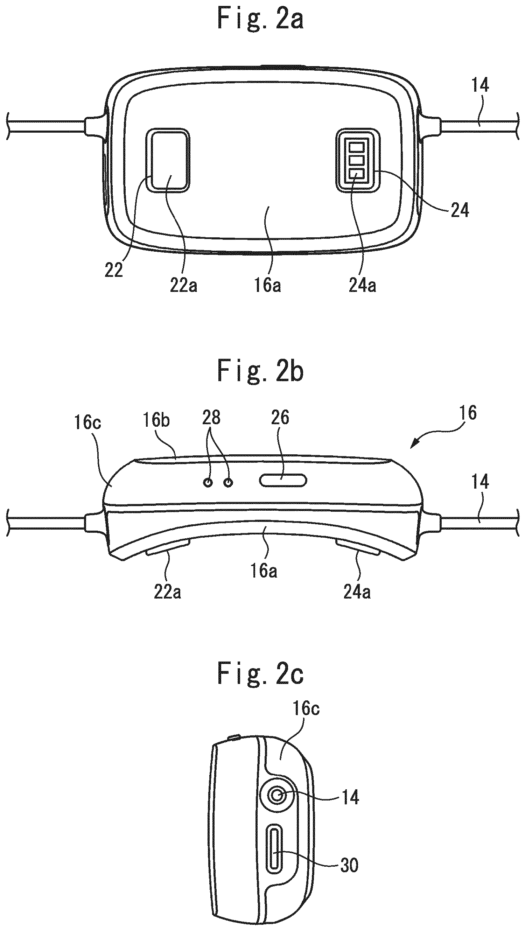

FIG. 2(a) to FIG. 2(c) respectively show a front view, plan view, and side view of the board housing 16. The board housing 16 extends from the side part 16c and is electrically connected with each earpiece housing 12 through wiring 38 bundled in a harness 14 (see FIG. 5(a)). The front part 16a of the board housing 16 is shaped curved along the back neck part. At the outer circumference of the board housing 16, a not shown sheet shaped attachment part is formed integrally with the board housing 16. For this reason, the board housing 16 can be stably attached to the back neck part of the user HP.

At the two left and right sides of the front part 16a of the board housing 16, a body temperature sensor 22 used as a bioinformation sensor for measuring the body temperature at the surface layer and deep parts of the user HP and a photoelectric sensor (PPG sensor) 24 used as a bioinformation sensor for measuring the pulse wave, blood pressure, and plasma glucose as bioinformation of the user HP are arranged with contact parts 22a, 24a for attachment to the body surface BS exposed. Further, at the top of the back part 16b side, a power switch 26 of the biosignal measurement device 10 and an LED 28 for displaying the operating mode are arranged. Further, at the side 16c side, a USB port 30 for sending the measured data to an outside apparatus is provided.

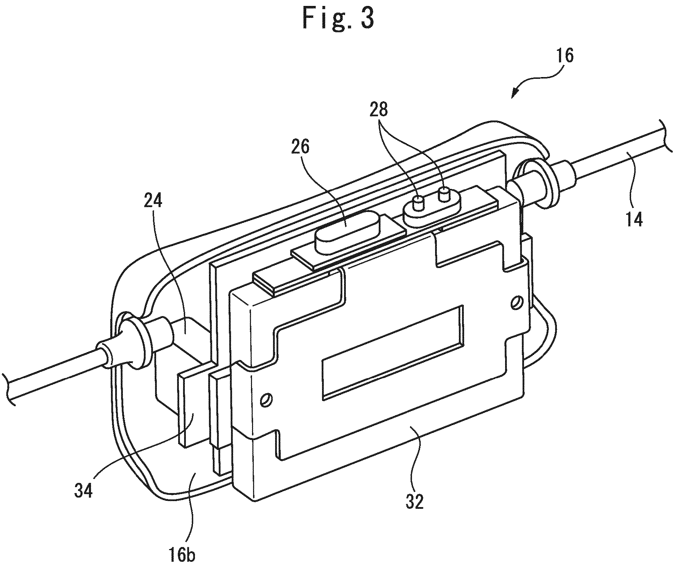

FIG. 3 is a back view of the board housing 16 in the state with the cover of the back part 16b removed. At the inside of the board housing 16, a battery 32 forming the power source of the various measurement sensors of the biosignal measurement device 10 and the board 34 are arranged. At the board 34, the electrodes 18 of the earpiece housing 12, body temperature sensor 22, and photoelectric sensor 24 are electrically connected. A not shown six-axis acceleration meter is arranged for measuring the median acceleration as bioinformation for obtaining a grasp of body movement. Further, at the board 34, an AD converter 36 (see FIG. 5A) and a memory (not shown) are arranged for recording the data measured by these measurement sensors such as an electroencephalogram, electrocardiogram, electromyogram, body temperature (surface layer and deep part), pulse wave, blood pressure, plasma glucose, and median acceleration. Note that, here, as bioinformation sensors, it was explained that a body temperature sensor 22 and photoelectric sensor 24 were arranged, but the invention is not limited to this. As bioinformation sensors, sensors or electrodes for measuring an electroencephalogram (EEG), electrocardiogram (ECG), and electromyogram (EMG) may be further provided.

Next, the action and effect of the biosignal measurement device 10 according to the present embodiment will be explained below.

According to the biosignal measurement device 10 according to the present embodiment, the earpiece housings 12 provided with the electrodes 18 and the board housing 16 having a photoelectric sensor 24 for acquiring a biosignal (bioinformation) other than the biopotential such as the pulse waves, blood pressure, and plasma glucose are attached to separate portions of the user HP. For analysis and evaluation of the biosignal, the biosignal has to be measured by a significant waveform. It is preferable to measure it at a portion close to the signal source. Here, the electrodes 18 and the photoelectric sensor 24 are separately attached at places close to the brain and heart such as the back sides of the auricles suitable for measuring the biopotential for an electromyogram and at the back neck part close to the carotid artery, so it is possible to suitably measure both the biopotential and bioinformation.

Furthermore, according to the biosignal measurement device 10 according to the present embodiment, each earpiece housing 12 is configured to be able to measure the bioimpedance. The earpiece housings 12 able to measure the biopotential generated due to activity of a biological being can also be used for measurement of the bioimpedance generated by electrically stimulating the biological being. For this reason, it is possible to prevent or keep down an unnecessary increase in the number of sensors and prevent or keep down the increase in weight of the earpiece housings 12. Due to this, it is possible to reduce the stress of the user HP wearing the biosignal measurement device 10 and improve the worn feeling.

Further, according to the biosignal measurement device 10 according to the present embodiment, the bioinformation acquisition sensor of the board housing 16 includes a photoelectric sensor 24. For this reason, it is possible to suitably measure bioinformation such as the blood pressure, plasma glucose, and pulse.

Furthermore, according to the biosignal measurement device 10 according to the present embodiment, the board housing 16 is configured to be able to measure the bioimpedance, temperature, and acceleration. For this reason, when analyzing and evaluating the measured biosignal, it is possible to obtain a suitable grasp of the correlation between the biosignal and the bioimpedance, temperature, and acceleration.

Further, according to the biosignal measurement device 10 according to the present embodiment, it is possible to provide sensors for measuring diverse biosignals dispersed at the earpiece housings 12 and board housing 16 attached to separate portions of the user HP. For this reason, it is possible to form the earpiece housings 12 and board housing 16 in smaller sizes and keep down the increase in weight. Due to this, it is possible to reduce the stress of the user HP wearing the biosignal measurement device 10 and improve the worn feeling.

As explained above, the biosignal measurement device 10 according to the present embodiment can perform measurement at suitable portions corresponding to the biosignal to be measured.

### (Second Embodiment)

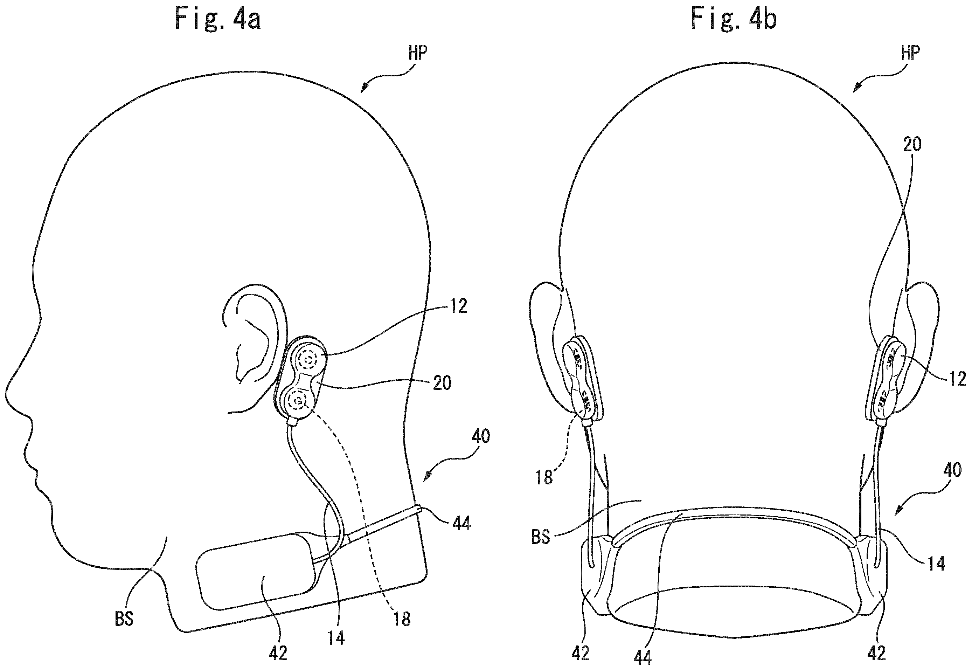

Below, using FIG. 4(a) to FIG. 5(c), a biosignal measurement device 40 according to a second embodiment will be explained. Elements similar or corresponding to FIG. 1 will be assigned the same notations and overlapping explanations will be omitted.

FIG. 4(a) and FIG. 4(b) are views, seen from the side and back side, of a biosignal measurement device 40 attached to a user HP. Here, one each board housing 42 used as the bioinformation acquiring part is attached to each of the side neck parts of the two left and right sides of the user HP. These are electrically connected to the left and right earpiece housings 12 through the harness 14. They are connected by a band 44 extending along the head at the back neck part side. The band 44 has elasticity and generates a spring force so as to pull the board housings 42 attached to the user HP toward the user HP. For this reason, it is possible to stabilize the positions of the board housings 42 attached to the user HP.

FIG. 5(a) to FIG. 5(c) respectively are a side view, plan view, and bottom view of each board housing 42. FIG. 5(b) is a side view of the state where the cover of the side part 42a at the side facing the user HP is removed. At the back side of the side part 42a of the board housing 42, an attachment part 46 is formed to closely contact the side neck part of the user HP. The body temperature sensor 22 and photoelectric sensor 24 are arranged so that the contact parts 22a, 24a for attachment to the body surface BS are exposed from the attachment part 46.

At the top 42b side of the board housing 42, a power switch 26 of the biosignal measurement device 10 and an LED 28 for displaying the operating mode are arranged. Further, at the bottom 42c side, a USB port 30 for sending the measured data to an outside apparatus is provided.

Inside each board housing 42, a battery (not shown) serving as the power source for the sensors of the biosignal measurement device 40 and a board 34 are arranged. At the board 34, electrodes 18 of the earpiece housing 12, a body temperature sensor 22, and a photoelectric sensor 24 are electrically connected and a not shown 6-axis accelerometer for measuring the median acceleration for obtaining a grasp of body movement is arranged. Further, at the board 34, an AD converter 36 (see FIG. 5(a)) and memory (not shown) are arranged for recording data measured by these sensors associated with an electroencephalogram, electrocardiogram, electromyogram, body temperature (surface layer and deep part), pulse wave, blood pressure, plasma glucose, and median acceleration. Further, the board 34 is provided with a chip 48 for data processing and communication processing. Note that, here, as the bioinformation sensors, it was explained that a body temperature sensor 22 and photoelectric sensor 24 were arranged, but the invention is not limited to this. As bioinformation sensors, sensors or electrodes for measuring an electroencephalogram (EEG), electrocardiogram (ECG), and electromyogram (EMG) may be further provided.

According to the biosignal measurement device 40 according to the present embodiment, the earpiece housings 12 provided with the electrodes 18 and the board housings 42 having the photoelectric sensors 24 for acquiring the pulse wave, blood pressure, and plasma glucose as biosignals other than the biopotential (bioinformation) are attached to separate portions of the user HP. Specifically, the electrodes 18 and the photoelectric sensors 24 are separately attached at parts close to the brain and the heart such as the back sides of the auricles and at the lateral neck part close to the carotid artery suitable for measurement of the biopotential relating to an electromyogram, so it is possible to suitably measure both the biopotential and bioinformation.

As explained above, the biosignal measurement device 40 according to the present embodiment enables measurement at a suitable portion corresponding to the biosignal to be measured.

Above, embodiments of biosignal measurement devices 10, 40 were explained, but the present invention is not limited to the above embodiments. A person skilled in the art, it is believed, can understand that the embodiments can be modified in various ways.

### REFERENCE SIGNS LIST

10. biosignal measurement device
12. earpiece housing (biopotential acquiring part)
16. board housing (bioinformation acquiring part)
18. electrode (biopotential sensor)
22. body temperature sensor (bioinformation sensor)
24. photoelectric sensor (bioinformation sensor)
40. biosignal measurement device
42. board housing (bioinformation acquiring part)
HP. user

## Claims

1. A biosignal measurement device to be worn by a user, the biosignal measurement device comprising
at least one biopotential acquiring part having a biopotential sensor and
a bioinformation acquiring part having a bioinformation acquisition sensor for acquiring a biosignal other than the biopotential as bioinformation,
the biopotential acquiring part and the bioinformation acquiring part being attached at separate portions of the user.

2. The biosignal measurement device according to claim 1, wherein the biopotential acquiring part is attached to at least one of the back side of an auricle, head, shoulder, and back of the user.

3. The biosignal measurement device according to claim 2, wherein the bioinformation acquiring part is attached to the back neck part of the user.

4. The biosignal measurement device according to claim 3, wherein the biopotential sensor is configured to be able to measure at least one of an electroencephalogram, electrocardiogram, and electromyogram of the user.

5. The biosignal measurement device according to claim 4, wherein the biopotential acquiring part is configured to be able to measure a bioimpedance.

6. The biosignal measurement device according to claim 5, wherein the bioinformation acquiring part is configured to be able to measure a blood pressure, plasma glucose, pulse, body surface temperature, and median acceleration.

7. The biosignal measurement device according to claim 6, wherein the bioinformation acquisition sensor includes a photoelectric sensor.

8. The biosignal measurement device according to claim 7, wherein the bioinformation acquiring part is configured to be able to measure at least one of a bioimpedance, temperature, and acceleration.
